# EUROPEAN PATENT SPECIFICATION

(11) **EP 1 010 423 B1**
(45) Date of publication and mention of the grant of the patent: **15.02.2006**
(21) Application number: 98932185.6
(22) Date of filing: 13.07.1998
(51) Int. Cl.: A61K 9/16, A61K 9/50

(54) **ORAL PHARMACEUTICAL PREPARATION COMPRISING AN ANTIULCER BENZIMIDAZOLE DERIVATIVE, AND PROCESS FOR ITS PRODUCTION**
ORALE PHARMAZEUTISCHE ZUSAMMENSETZUNG ENTHALTEND EIN BENZIMIDAZOLDERIVAT MIT ANTIULCUS WIRKUNG UND VERFAHREN ZU SEINER HERSTELLUNG
PREPARATION PHARMACEUTIQUE ORALE RENFERMANT UN DERIVE DE BENZIMIDAZOLE A ACTIVITE ANTI-ULCEREUSE ET PROCEDE D'OBTENTION

(30) Priority: 31.07.1997 ES 9701816
(43) Date of publication of application: 21.06.2000
(73) Proprietor: Liconsa, Liberacion Controlada de Sustancias Activas, S.A., 08028 Barcelona (ES)
(72) Inventor: PICORNELL DARDER, Carlos, E-28043 Madrid (ES)
(74) Representative: Ponti Sales, Adelaida
(86) International application number: PCT/ES1998/000204
(87) International publication number: WO 1999/006032

(56) References cited:
- EP-A- 0 519 144
- EP-A- 0 519 365
- EP-A- 0 642 797
- EP-A- 0 773 025
- WO-A-93/25204
- WO-A-96/01624
- WO-A-97/02020

## Description

### Field of the invention

The present invention relates co new pharmaceutical formulation for oral administration which includes a compound of anti-ulcer activity, and to a procedure for making same.

### Background of the invention

In recent times numerous techniques have been developed for preparing systems of release in the form of microgranules. In them, the mixture of active ingredient and excipients can be submitted to a process of kneading, extrusion, spheronization, coating, etc. Each of these pelletization techniques calls for a different technology, so that there are many types of pelletization equipment: coating pans or drums, fluid-bed equipment, extruders-spheronizers and centrifuging equipment, among others. The final result would appear to be the same, although there are in fact considerable differences between the pellets made using each technique.

Various types of microgranules have been described for the formulation of certain benzimidazoles with anti-ulcer activity, such as those of European patents EP 247983, EP 244380, EP 237200 and. EP 277741 and international patent WO 92/22284. This type of compounds are in general acid-labile and for that reason various procedures have been developed to protect them from the effect of the gastric acid medium.

In European patents EP 247983 and EP 244380 the active ingredient is kneaded by wet process with a mixture of excipients which allows an alkaline microenvironment to be created. The mixture is extruded and then spheronized. The spheronized microgranules are coated with one or more intermediate layers of excipients soluble in water, alkalis, buffer solutions, polymeric solutions, etc., and an external gastro-resistant layer is then applied.

As this is an extrusion-spheronization method, the total yield of the process will depend upon many factors. On the one hand, during the extrusion phase it is essential to control dimensions such as the cross-section and the length of the extrudate so as to avoid great dispersion in the size and shape of the particles. Both factors would explain the subsequent coating being irregular and would even lead to the presence of pores, unless an excess quantity were projected in order to ensure complete coating of the microgranule, though this would in turn cause problems when it came to standardizing release of the active ingredient. On the other hand, the characteristics of cohesiveness, firmness and plasticity of the extrudate must be controlled if its subsequent spheronization is to be ensured.

To these problems is added the fact that the need to use several pieces of equipment such as kneading machines, extruding machines and spheronizers means that losses through kneading, extrusion and spheronization can be greater than with other pelletization methods.

European patents EP 237200 and EP 277741, this last published in Spain as ES 2.052.697, show an example of coating with sprinkled powder (powder-layering) using a rotogranulating machine. Spherical granules are described which have a nucleus coated with dusted powder which contains an anti-ulcer benzimidazolic compound and hydroxypropyl cellulose with low degree of replacement. Also described is a procedure for producing the aforesaid spherical granules, characterized in that the seeding nuclei are wetted by spraying thereof with an agglutinant solution and they are dusted with a powder which contains the active ingredient and the hydroxypropyl cellulose little replaced.

The technique of coating using a rotogranulating machine is very abrasive, especially in the initial phase of the process. Apart from abrasion of the particles against the walls of the machine due to the thrust of the air, a situation normal in any fluid bed, there is a shear force exercised by the rotary disc of the rotogranulating machine. All this often leads to problems such as breakage and abrasion of the granules.

These problems not only make control of the release of active ingredient more difficult, but also have a considerable effect on granule production output. For this reason, and in order to reduce these problems, European patent EP 277741 proposes as a solution the use of extremely hard seeding nuclei.

For the preparation of the aforesaid spherical granules, European patent 277741 describes the use of rotogranulating machine of centrifugal type such as the CF360 rotogranulating machine by Freund Co. In this procedure, two layers are added successively, though leaving them perfectly separate. In the first, the active ingredient is added with excipients in powder form simultaneously with a solution of the aqueous binder. In the second, the excipients are simply added in powder form along with the aqueous binder solution. The procedure of addition of the active layer according to EP 277741 means that the layer is quite porous and is distributed in a manner which is not perfectly uniform over the surface of the initial inert particle.

The spherical granules obtained are dried for sixteen hours and then passed through a cascade of sieves in order to select the best range of sizes. Finally, to apply the enteric coating, the dry sieved granules are placed in a "Wurster" type fluid bed. In short, the spherical granules with gastro-resistant coating described in European patent EP 277741 have passed through four different pieces of equipment.

Further examples of coating of pellets using the dry powder layering technique are disclosed in patents EP 642 797 and WO 93 25204. In european patent EP 642 797, lansoprazole pellets comprising a protective, separating inert reacting layer are described. In order to produce said pellets, several pieces of equipment are used for each step of the process: a centrifugal fluidized bed granulator for the active coating, a fluidized bed coator for the enteric coaling and a vacuum for the drying steps.

In patent WO 93 25204, omeoprazole pellets bearing a separating inert reacting layer are produced using a procedure which implies as well the use of several pieces of equipment.

### Description of the invention

In the present invention a formulation and a working methodology in a fluid bed of the "Wurster" type or the like have been developed. In it, the negative factors which affected the methods described to date are eliminated and substantial changes introduced with respect to the methods of previous patents for pellets containing benzimidazoles.

The object of the present invention is to find new pharmaceutical formulations for the oral administration of anti-ulcer active ingredients of the benzimidazole formula I type 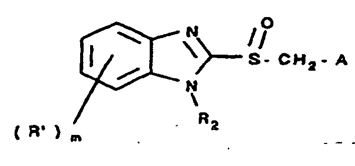
in which:
A can be:

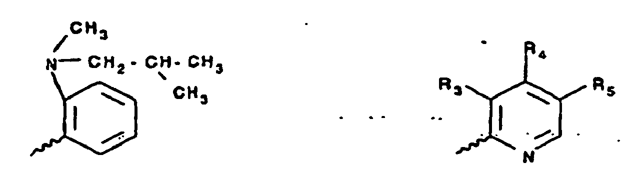

in which: R³ and R⁵ are the same or different, and can be hydrogen, alkyl, alkoxy, or alkoxyalkoxy; and
R⁴ is hydrogen, alkyl, alkoxy which can optionally be fluorinated alkoxyalkoxy, or alkoxycycloalkyl;
R¹ is hydrogen, alkyl, halogen, cyano, carboxy, carboalkoxy, carboalkoxyalkyl, carbamoyl, carbamoylalkyl, hydroxy, alkoxy, hydroxyalkyl, trifluoromethyl, acyl, carbamoyloxy, nitro, acyloxy, aryl, aryloxy, alkylthio or alkylsulfinyl;
R² is hydrogen, alkyl, acyl, carboalkoxy, carbamoyl, alkylcarbamoyl, dialkylcarbamoyl, alkylcarbonylmethyl, alkoxycarbonylmethyl or alkylsulfonyl; and, m is a whole number from 0 to 4;
or of formula II or III: 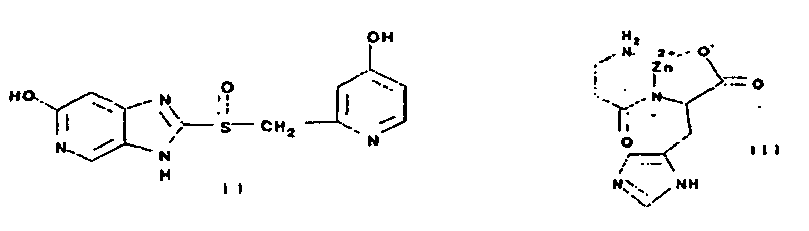
hereinafter generally denominated anti-ulcer compounds.

The new galenical formulations object of the present invention are characterized in that they are spherical granules with a homogeneous active charge layer and a very unporous surface, formed by coating of an inert nucleus by spraying a single aqueous or hydroalcoholic mixture containing the active ingredient (anti-ulcer compound) together with the other excipients. Then, in the same equipment and following a short drying period, the granules obtained are subjected to a stage of enteric coating. Optionally, if it is desired to obtain lower humidity, recourse can be had to additional drying.

Said formulations resolve satisfactorily and innovatively the difficulties described in the prior state of the art, while at the same time showing resistance to dissolution in acid medium (gastro-resistant) and dissolving rapidly in alkaline medium with disintegration of the granules and excellent release of active ingredient.

The present invention satisfactorily resolves the difficulty involved in coating the inert nucleus with an aqueous or hydroalcoholic solution suspension containing a un anti-ulcer compound which is generally highly labile in an acid environment or environment and in aqueous dissolution, in the presence of disintegrating-swelling excipients which cause an increase of viscosity which enormously hinders spraying thereof onto the inert nuclei.

El "Wurster" type fluid bed or the like in which the coating process is carried out minimizes the abrasion caused by rotogranulation. It is therefore unnecessary to use a especially hard inert nucleus.

The microgranule is not subjected to any kneading or extrusion process, nor is an inert nucleus coat sprinkled with powder dusted together with an aqueous binder. The microgranule used in the present invention consists in an inert nucleus which is coated with a single active layer made up of an aqueous or hydroalcoholic suspension-solution which includes the anti-ulcer component and at least one disintegrating-swelling excipient, a binder, an alkalizing medium, a surface-active agent and a diluent.

When a single suspension-solution is projected onto the inert nucleus, a less porous and more homogeneous product is obtained than in the procedures known to date, and all the subsequent operations are simplified considerably.

Likewise, unlike what happened in the prior art (EP 244.380, EP 277.983, EP 237.200, EP 277.741, PCT WO92/22289), in which the manufacturing procedure was carried out using several different pieces of equipment, in the present invention the entire process is carried out using a single piece of fluid-bed equipment, thereby 5 minimizing losses of time and of product, while more easily complying with Good Manufacturing Practice (GMP) for medicaments. What is more, avoidance of handling and intermediate steps considerably reduces the investment required in machinery and buildings.

The inert nuclei used are microspherical neutral granules which can have in their composition two or more of the following substances: sorbitol, mannitol, saccharose, starch, microcrystalline cellulose, lactose, glucose, trehalose, maltitol and fructose. The initial size of same can be between 200 and 1800 micrometres, preferably between 600-900 micrometres.

The single aqueous or hydroalcoholic solution-suspension which is sprayed onto the inert nucleus is made up of the active ingredient with anti-ulcer activity and the other excipients. The hydroalcoholic medium is made up of mixtures of water:ethanol in proportions less than or equal to 50% v/v, preferably between 25%-45% v/v.

The oral pharmaceutical preparation obtainable by a process as defined later includes a compound with anti-ulcer activity as its active ingredient and is characterized in that it also includes:
a) un inert nucleus;
b) a soluble active layer or layer which disintegrates rapidly in water, made from a single aqueous or hydroalcoholic solution-suspension which includes:
   - an active ingredient of anti-ulcer activity of general formula I

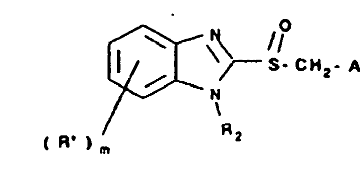

   in which:
   A can be: 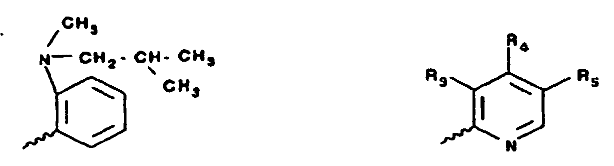
      in which: R³ and R⁵ are the same or different, and may be hydrogen, alkyl, alkoxy, or alkoxyalkoxy; and
   R⁴ is hydrogen, alkyl, alkoxy which can optionally be fluorinated, alkoxyalkoxy, or alkoxycycloalkyl;
   R¹ is hydrogen, alkyl, halogen, cyano, carboxy, carboalkoxy, carboalkoxyalkyl, carbamoyl, carbamoylalkyl, hydroxy, alkoxy, hydroxyalkyl, trifluoromethyl, acyl, carbamoyloxy, nitro, acyloxy, aryl, aryloxy, alkylthio or alkylsulfinyl;
   R² is hydrogen, alkyl, acyl, carboalkoxy, carbamoyl, alkylcarbamoyl, dialkylcarbamoyl, alkylcarbonylmethyl, alkoxycarbonylmethyl or alkylsulfonyl; and, m is a whole number from 0 to 4;

   or of formula II or III, 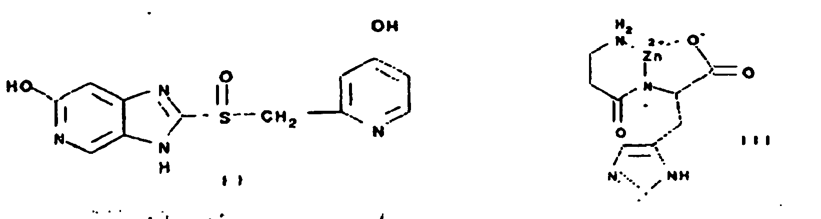
   - an alkaline reacting compound and
   - at least one pharmaceutically acceptable excipient selected from the group which includes: a binder, an alkaline reacting compound, a surface-active agent, a filling material and a disintegrating-swelling excipient; and
c) a gastro-resistant outer coating made from a solution which includes:
   - an enteric coating polymer; and
   - at least one excipient chosen from the group which includes: a plasticizer, a surface-active agent, a pigment and a lubricant. .

The alkaline reaction compound may be selected from a compound with alkaline reaction, such as trisodium and disodium phosphate, the oxide, hydroxide or carbonate of magnesium, hydroxide of aluminium, carbonate, phosphate or citrate of aluminium, calcium, sodium or potassium, the mixed compounds of aluminium/magnesium Al₂O₃.6MgO.CO₂.12H₂O or MgO.Al₂O₃.2SiO₂.nH₂O or similar compounds and amino acids with alkaline reaction.

Among the excipients present in the suspension-solution of the active compound of formula I,II or III which is sprayed onto the inert nuclei are:
a) a binder or mixture of binders: saccharose, starch, methyl cellulose, carboxymethyl cellulose (CMC), hydroxypropyl cellulose (HPC), hydroxypropylmethyl cellulose (HPMC), polyvinyl pyrrolidone (PVP), dextrine or gum arabic, dissolved in water, ethanol, or a mixture of both (50% v/v or less).
b) a surface-active agent, such as sodium lauryl sulphate, polysorbate, poloxamer and ionic and non-ionic surface-active agents.
c) a filling material such as lactose, starch, saccharose or microcrystalline cellulose
d) a disintegrating-swelling compound, such as starch, calcium carboxymethyl cellulose (CMCCa), sodium glycolate starch or hydroxypropyl cellulose (L-HPC).

Once the microgranules have been formed by spraying the aqueous or hydroalcoholic suspension-solution containing the active ingredient, they are dried and coated with a layer of the enteric coating.

The following can be used as enteric coating polymers: methyl cellulose, hydroxyethyl cellulose (HEC), hydroxybutyl cellulose (HBC), HPMC, ethyl cellulose, hydroxymethyl cellulose (HMC), HPC, polyoxyethylene glycol, castor oil, cellulose phthalic acetate, phthalate of HPMC, succinate acetate of HMC, sodium carboxymethylamylopectin, chitosan, alginic acid, carrageenans, galactomannons, tragacanth, shellac, agar-agar, gum arabic, guar gum and xanthan gum, polyacrylic acids, methacrylics and their salts, polyvinyl alcohol (PVA), polyethylene and polyproprylene oxides and mixtures thereof. The gastro-resistant polymer can be accompanied by: plasticizers such as triethylcitrate (TEC), polyethylene glycol (PEG), cetyl and stearyl alcohol; surface-active agents such as sodium lauryl sulphate, polysorbate and poloxamer; pigments such as titanium dioxide, iron sesquioxide; lubricants such as talc, magnesium stearate or glyceryl monostearate, together with a mixture of same.

Another object of the present invention is a manufacturing procedure for said galenical formulations.

The procedure for obtaining the oral pharmaceutical preparation of the invention is according to claim 1.

Optionally, after stage 3) of coating of the charged nuclei, an additional drying is carried out.

There follows a description of the procedure of the invention, with special reference to the method and percentages used for each of the components.

In a tank of suitable dimensions an alkaline aqueous or hydroalcoholic solution is prepared by incorporating the alkaline-reaction compound into the aqueous or hydroalcoholic vehicle in a percentage of between 0.1%-5% (p/p). Using continuous agitation, the anti-ulcer benzimidazolic compound and another compound with anti-ulcer activity (6%-25% p/p) are incorporated together with the filler material (3-15% p/p). To the suspension-solution obtained is added the surface-active agent (0.01%-3% p/p), a binder and a disintegrating-swelling agent in percentages of between 2%-10% respectively, taking account of the times of use of the prepared solution.

Homogenization of the mixture is carried out with continuous agitation and at ambient temperature (23 ± 2°C). Agitation is maintained during the spraying phase of the active layer on the inert pellets; this process is carried out using a "Wurster" type fluid bed or similar equipment, into which the inert nuclei of size 850µm are poured. The spraying conditions are as follows: Spraying pressure: 2-3bar. Product temperature: 35-45°C. Volume of air: 700-1200m³/h at 80-90°C. Nozzle diameter:1.2 mm).

Once the charging phase has been completed, the nuclei coated with the active ingredient are dried in the same equipment. The air flow is 600-800 m³/h at temperature of 35-45 °C for 45 minutes.

The next stage is enteric coating of the active pellets, which is carried out in the same equipment. An aqueous or organic dispersion of the gastro-resistant polymer (10-40% p/p) is prepared. The plasticizer (0.2-10% p/p) is in turn dissolved in water and the surface-active agent added with constant agitation (up to 3% p/p) and, where necessary, pigments (0-5% p/p) and lubricants (0.5-16% p/p). Once the mixture has been homogenized the dispersion of the gastro-resistant polymer (25-45% p/p) is added whilst agitating.

In order to obtain lower humidity content, an additional drying can be carried out using a conventional dryer.

Over 90% of the resulting microgranules must be of a diameter between 0.4 and 1.95 mm, and more specifically between 0.5-1.8 mm.

The nuclei object of the present invention are resistant to dissolution in acid medium, dissolve rapidly in alkaline medium, are stable over long storage periods, have excellent disintegration characteristics, and the active layer is more homogeneous and less porous than the granules described in the previous patents.

The present invention resolves satisfactorily the disadvantages deriving from the prior art, since a single suspension-solution is prepared for charging the inert nuclei. For this phase a fluid bed of the Wurster type or the like is used, this being much less abrasive than the rotogranulating machine which has to be used when a seeding nucleus is coated with an active powder and a binder solution.

From the time that charging of the inert nuclei starts until the enteric coating is completed, the entire procedure is carried out on a single "Wurster" type fluid bed or the like, unlike other procedures which take place on several different pieces of equipment.

### Brief description of the figures

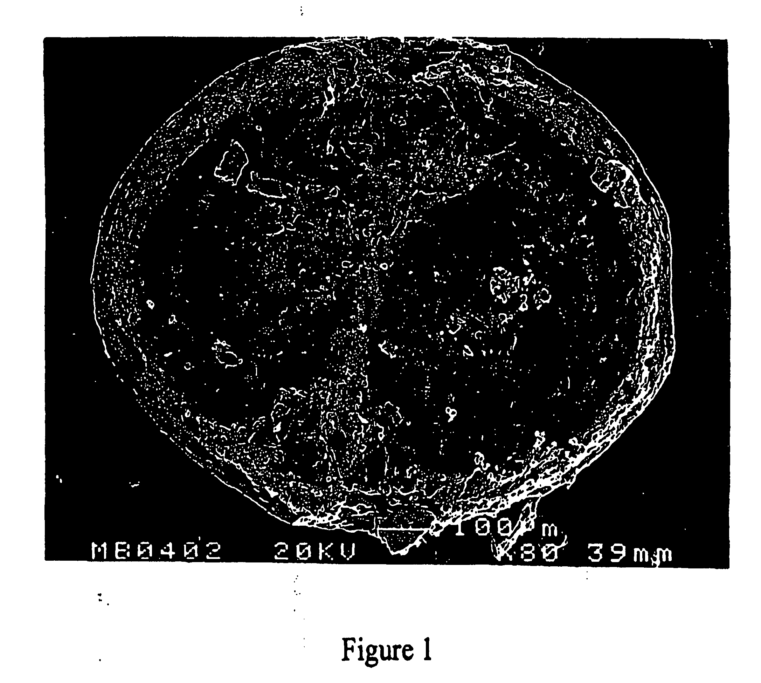

Figure 1 is a photograph obtained by electron microscope scanning, showing a section of the lansoprazole pellet of example 1.

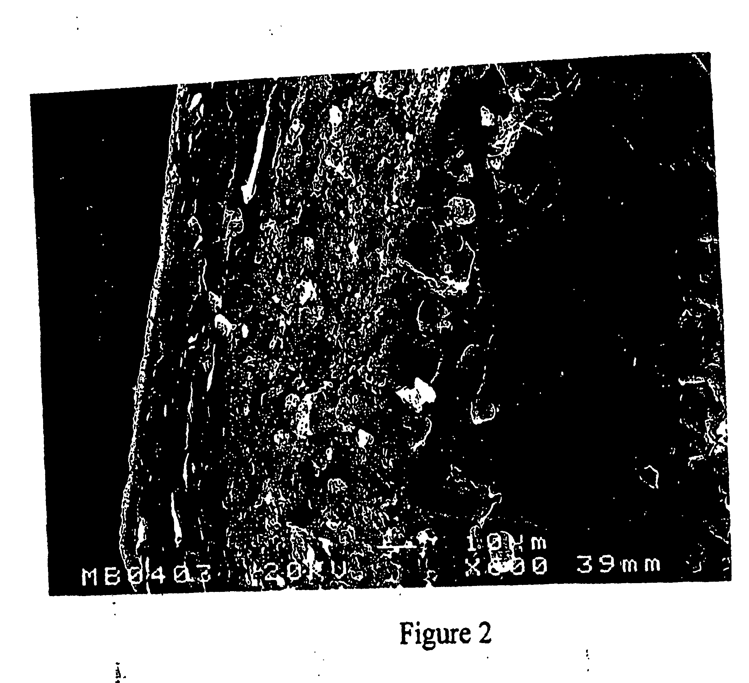

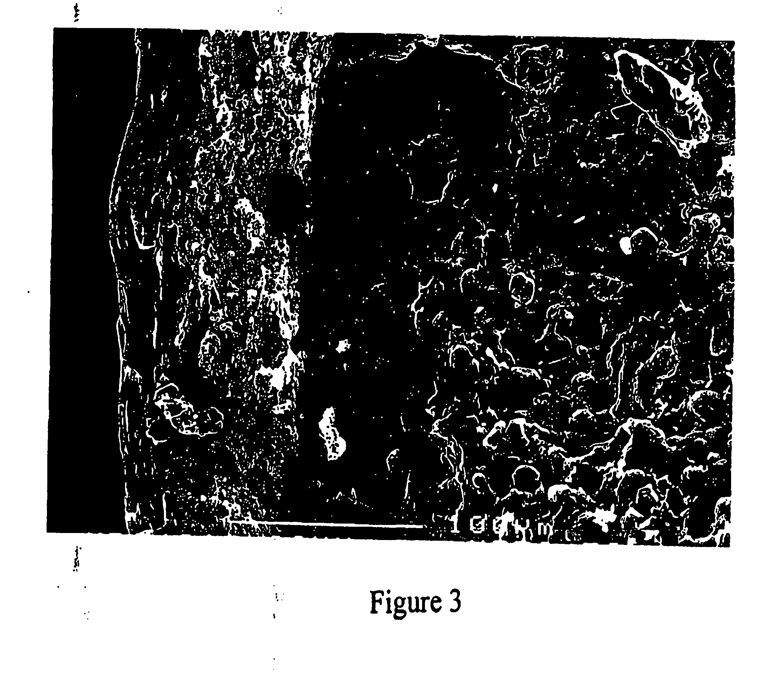

Figures 2 and 3, are photographs also obtained by electron microscopy, showing further details of the layers present.

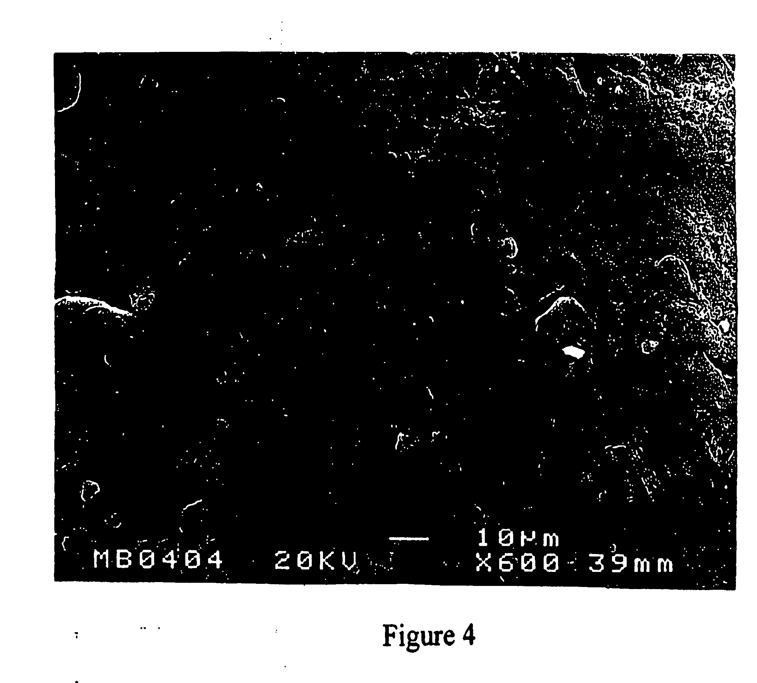

Figure 4 is a photograph showing the porosity of the coating.

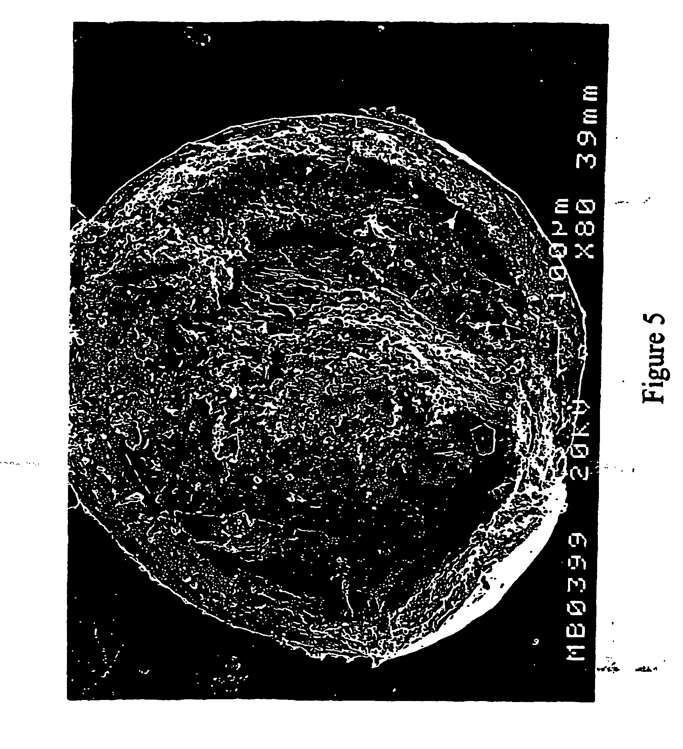

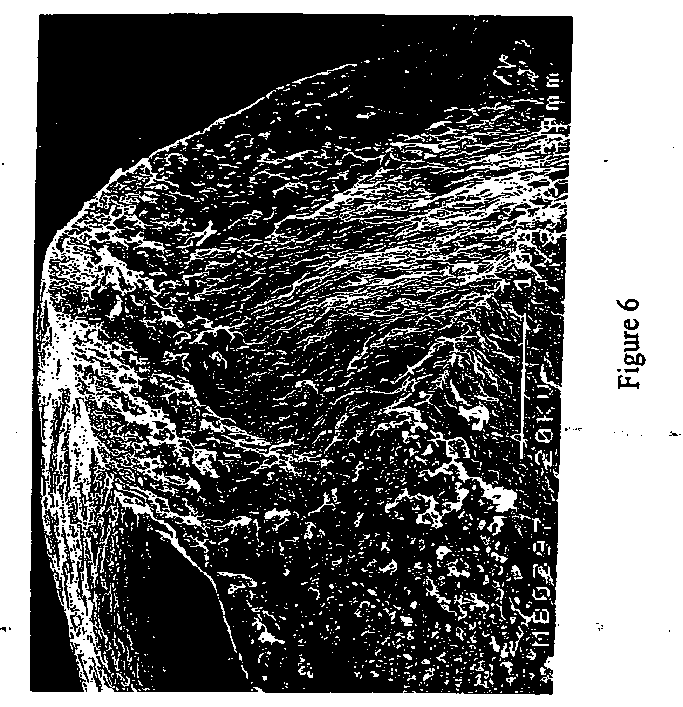

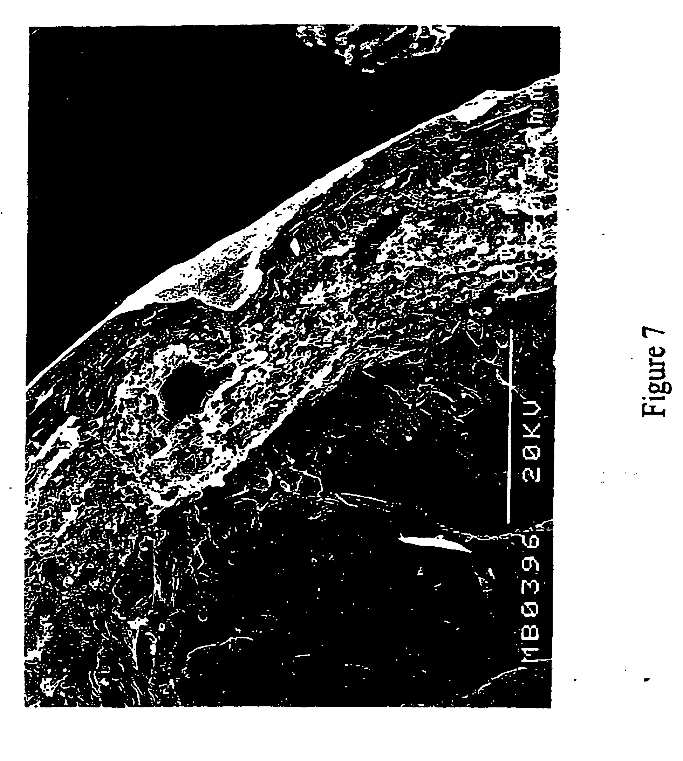

Figures 5, 6 and 7, are photographs showing a section of the omeprazole pellet of example 2 with gastro-resistant coating of formula I.

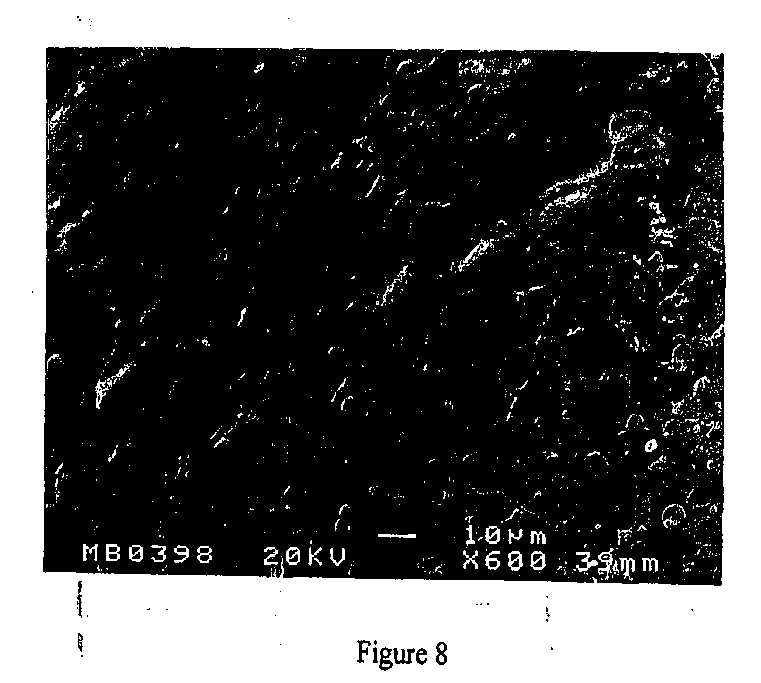

Figure 8 is a photograph showing the homogeneity of the coating and the few pores of same.

### EXAMPLES

For a better understanding of all that has been set out above, some examples are provided which, schematically and solely by way of non-restrictive example, show a practical example of embodiment of the invention.

### EXAMPLE 1

In a stainless steel receptacle of sufficient capacity an alkalizing aqueous solution of trisodium phosphate was prepared, and to this were added lansoprazole lactose and sodium lauryl sulphate, with continuous agitation throughout. When the mixture was homogeneous the colloidal aqueous solution of hydroxypropylmethyl cellulose (13.50% p/p) was added, maintaining agitation in order to ensure homogeneity of the product. L-HPC was then incorporated into that solution-suspension. Agitation was maintained up till the moment of spraying onto the neutral pellets.

| | |
|---|---|
| Lansoprazole | 1.29 Kg |
| Sodium lauryl sulphate | 5.28 10⁻¹ Kg |
| Crystallized disodium phosphate | 0.052 Kg |
| Hydroxypropylmethyl cellulose | 0.8 Kg |
| Lactose | 0.51 Kg |
| Hydroxypropyl cellulose | 0.39 Kg |
| Water | 14.28 Kg |

10 kg of inert nuclei were incorporated, made up of saccharose (62.5-91.5 %) and starch (37.5-8.5 %) of 800 micrometres average size in a NIRO "Wurster" type fluid bed and was covered with the solution-suspension prepared in advance, under the following conditions: air flow: 250m³/hora. Diameter of nozzles: 1.2 mm. Spraying pressure: 2.5 bar. Spraying of product: 100 g/min. Air temperature: 85°C. Product temperature: 38°C.

The charged nuclei were then dried in the same bed for 45 minutes with air at a temperature of 35°C and with an air flow of 250m^{3/}h in order to obtain a suitable degree of humidity.

The dry granules were subjected to enteric coating by spraying the gastro-resistant solution-suspension detailed below and prepared from an aqueous solution of polyethylene glycol into which were incorporated the other excipients, with continuous agitation

| | |
|---|---|
| Talc | 0.57 Kg |
| Titanium dioxide | 0.18 Kg |
| Polyethylene glycol 6000 | 0.18 Kg |
| Polysorbate | 0.08 Kg |
| Eudragit L30D55 | 5.78 Kg |
| Water | 12.14 Kg. |

The working conditions were as follows: air flow: 250 m³/hour. Diameter of nozzles: 1.2 mm. Spraying pressure: 2.5 bar. Spraying of product: 100g/min. Air temperature: 70°C. Product temperature: 36°C

Optional drying of the coated pellets was carried out for 45 minutes with air at a temperature of 35°C and with an air flow of 250m^{3/}h.

Set out below are the results of the stability studies carried out on a batch of Lansoprazole pellets under different storage conditions: ambient temperature, and 40°C and relative humidity 75%.

| Storage conditions: Ambient temperature Container: Topaz glass bottle with bag of silica gel inside fitted with metallic screw-threaded top including zelelastic seal | | | | | | |
|---|---|---|---|---|---|---|
| Test time | Colour | Gastroresistence | Release | Active Ing. | Humidity | Transmittance at 440nm |
| zero hour | cream white | 98.8% | 82.8% | 33.0mg/370mg | 1.62% | 97% |
| 1 month | cream white | 98.6% | 82.0% | 33.0mg/370mg | 1.50% | 97% |
| 3 months | cream white | 97.0% | 30.9% | 32.8mg/370mg | 1.48% | 97% |
| 6 months | cream white | 97.4% | 79.8% | 32.0mg/370mg | 1.47% | 96% |
| 18 months | cream white | 97.4% | 78.9% | 31.9mg/370mg | 1.46% | 95% |

| Storage conditions: Temperature: 40°C, 75% of humidity Container: Topaz glass bottle with bag of silica gel inside fitted with metallic screw-threaded top including zelelastic seal | | | | | | |
|---|---|---|---|---|---|---|
| Test time | Colour | Gastroresistance | Releas e | Active log. | Humidity | Transmittance at 440nm |
| zero hour | cream white | 98.8% | 82.8% | 33.0mg/370mg | 1.62% | 97% |
| 1 month | cream white | 97.8% | 81.2% | 32.0mg/370mg | 0.90% | 95% |
| 3 months | cream white | 97.6% | 80.8% | 31.8mg/370mg | 1.27% | 93% |
| 6 months | cream white | 96.9% | 79.8% | 31.2mg/370mg | 1.32% | 92% |

No significant differences were found in the values for gastro-resistance and release of active ingredient with respect to the initial values, independently of the storage conditions. Both tests were carried out according to Farmacopocia USP XXIII.
The power of active ingredient was determined by high-resolution liquid chromatography. The degradation products were evaluated on the basis of the transmittance results detected at 440nm.

From the results obtained it can be deduced that that there were no great differences with respect to the initial values. A slight loss of activity could be detected at six month's storage at a temperature of 40°C, which would explain the reduction of transmittance values at 440nm.

The results obtained show the chemical stability of the active ingredient under the storage conditions tested. Moreover, no considerable variations in the humidity of the pellets were detected during storage, thus showing the physical stability of the formulation.

All these results show the stability of the formulations object of the present invention, which are moreover different from those described in the prior art in that they have no intermediate separating layer between the active layer and the gastro-resistant layer.

The electron scanning microscopy study was carried out using a Jeol JSM6400 scanning microscope. Photograph number 1 shows a section of the pellet of lansoprazole of example 1, showing clearly the presence of the inert nucleus, the active layer, intimately linked to the nucleus, and the gastro-resistant coating. Photographs numbers 2 and 3 show further details of both layers more clearly, revealing the absence of an intermediate separating layer between them. Photograph number 4 shows the low porosity of the coating. The lack of surface pores explains the physical-chemical stability of the pellet.

### EXAMPLE 2

In a stainless steel receptacle the alkalizing aqueous solution of disodium phosphate was prepared, and to this were added the omeprazole, lactose and sodium lauryl sulphate. Agitation was maintained to total homogeneity and the colloidal solution of hydroxypropylmethyl cellulose (12.55% p/p) and hydroxypropyl cellulose (L-HPC) added. Agitation was maintained up till the moment of spraying onto the neutral pellets.

The qualitative-quantitative composition of the solution-suspension was as follows:

| | |
|---|---|
| Omeprazole | 1.38 Kg |
| Sodium lauryl sulphate | 5.28 10⁻³ Kg |
| Crystallized disodium phosphate | 0.052 Kg |
| Hydroxypropylmethyl cellulose | 0.68 Kg |
| Lactose | 0.51 Kg |
| Hydroxypropyl cellulose | 0.39 Kg |
| Water | 14.28 Kg |

10 kg of inert nuclei was incorporated, made up of saccharose (62.5-91.5 %) and starch (37.5-8.5 %) of 800 micrometres average size in a NIRO "Wurster" type fluid bed and was covered with the solution-suspension prepared in advance, under the following conditions: air flow: 250 m³/hora. Diameter of nozzles: 1.2 mm. Spraying pressure: 2.5 bar. Spraying of product: 100 g/min. Air temperature: 75°C. Product temperature: 35°C.

The charged nuclei were then dried in order to obtain a suitable degree of humidity in the bed for 30 minutes with air at a temperature of 35°C and with air flow of 250 m³/h.

The dry granules were then subjected to enteric coating by spraying any of the gastro-resistant formulae shown below, prepared from the aqueous solution of polyethylene glycol to which were incorporated the other excipients under continuous agitation (Formula I) or from the organic solution of acetone and ethyl alcohol to which were incorporated the other excipients under continuous agitation (Formula II).

| Formula I | |
|---|---|
| Talc | 0.57 Kg g |
| Titanium dioxide | 0.18 Kg |
| Polyethylene glycol 6000 | 0.18 Kg |
| Polysorbate | 0.08 Kg |
| Eudragit L30D55 | 5.78 Kg |
| Water | 12.14 Kg. |

| Formula II | |
|---|---|
| Acetone | 20.86 Kg |
| Hydroxypropylmethyl cellulose phthalate | 2.35 Kg |
| Diethyl phthalate | 0.011 Kg |
| Ethyl alcohol | 8.93 Kg |

For this purpose, work was carried out under the following conditions: air flow: 250 m³/hour Diameter of nozzles: 1.2 mm. Spraying pressure: 2.5 bar-Spraying of product: 100 g/min. Air temperature: 70°C. Product temperature: 36°C.

The coated pellets were dried for 45 minutes with air at a temperature of 35°C and with a flow of 250m^{3/}h.

Below are set out the results of the stability studies carried out on a batch of omeprazole under different storage conditions: ambient temperature, and 30°C and relative humidity 65%.

| Storage conditions: Ambient temperature Container: Topaz glass bottle with bag of silica gel inside fitted with metallic screw-threaded top including zelelastic seal | | | | | | |
|---|---|---|---|---|---|---|
| Test time | Colour | Gastro-resistance | Release | Active Ing. | Humidity | Transmittance at 440nm |
| zero hour | cream white | 99.0% | 94.0% | 20.4mg/233mg | 1.12% | 98% |
| 1 month | cream white | 99.6% | 93.7% | 20.5mg/233mg | 1.14% | 98% |
| 3 months | cream vbite | 98.9% | 93.5% | 20.6mg/233mg | 1.20% | 98% |
| 6 months | cream white | 98.6% | 93.0% | 20.3mg/233mg | 1.25% | 98% |
| 18 months | cream white | 97.4% | 91.0% | 20.2mg/233mg | 1.35% | 96% |

| Storage conditions: Temperature: 30°C. Humidity: 65% Container: Topaz glass bottle with bag of silica gel inside fitted with metallic screw-threaded top including zelelastic seal | | | | | | |
|---|---|---|---|---|---|---|
| Test time | Colour | Gastroresistance | Release | Active Ing. | Humidity | Transmittance at 440nm |
| zero hour | cream white | 99.0% | 94.0% | 20.4mg/233mg | 1.12% | 98% |
| 1 month | cream white | 98.0% | 93.8% | 20.0mg/233mg | 1.16% | 97% |
| 3 months | cream white | 97.8% | 93.1% | 20.5mg/233mg | 1.26% | 96% |
| 6 months | cream white | 97.0% | 92.6% | 20.3mg/233mg | 1.37% | 95% |

The gastro-resistance, humidity and release values explain the physical stability of the pellet under the storage conditions tested. For their part, the power of the active ingredient and the transmittance values at 440nm ensure the chemical stability of the formulation.

All these results show the stability of the formulations object of the present invention, which moreover differ from those described in the prior art in that they have no intermediate separating layer between the active layer and the gastro-resistant layer.

The electron scanning microscopy study was carried out using a Jeol JSM6400 scanning microscope. Photographs numbers 5, 6 and 7 show a section of the pellet of omeprazole of example 2 with gastro-resistant coating of formula I, clearly showing the presence of the inert nucleus, the active layer, intimately linked to the nucleus, and the gastro-resistant coating. Photograph number 8 shows the homogeneity of the coating and the low number of pores, factors which enhance the physical stability of the pellet.

## Claims

1. Procedure for making an oral pharmaceutical preparation consisting of
a) an inert nucleus;
b) a soluble active layer or layer which disintegrates rapidly in water, made from a single aqueous or hydroalcoholic solution-suspension which includes:
- an active ingredient of anti-ulcer activity of general formula I 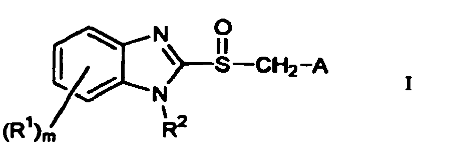
in which:
A can be: 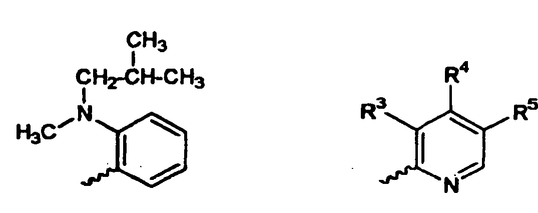
wherein:
R³ and R⁵ are the same or different, and may be hydrogen, alkyl, alkoxy, or alkoxyalkoxy;
R⁴ is hydrogen, alkyl, alkoxy which can optionally be fluorinated, alkoxyalkoxy, or alkoxycycloalkyl;
R¹ is hydrogen, alkyl, halogen, cyano, carboxy, carboalkoxy, carboalkoxyalkyl, carbamoyl, carbamoylalkyl, hydroxy, alkoxy, hydroxyalkyl, trifluoromethyl, acyl, carbamoyloxy, nitro, acyloxy, aryl, aryloxy, alkylthio or alkylsulfinyl;
R² is hydrogen, alkyl, acyl, carboalkoxy, carbamoyl, alkylcarbamoyl, dialkylcarbamoyl, alkylcarbonylmethyl, alkoxycarbonylmethyl or alkylsulfonyl; and
m is an integer from 0 a 4;
or of formula II or III, 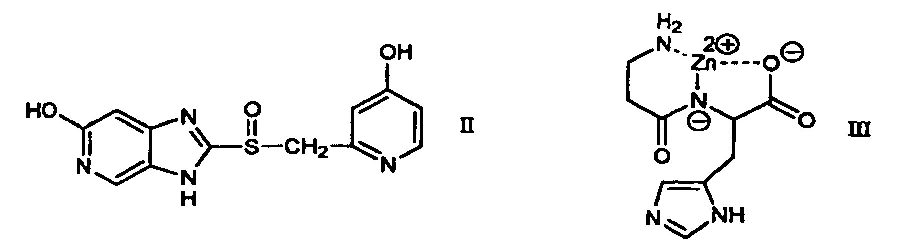
- an alkaline reacting compound and
- at least one pharmaceutically acceptable excipient selected from the group which includes: a binder, a surface-active agent, a filling material and a disintegrating-swelling excipient;
and
c) a gastro-resistant outer coating made from a solution which includes:
- an enteric coating polymer; and
- at least one excipient chosen from the group which includes: a plasticizer, a surface-active agent, a pigment and a lubricant,
being the procedure **characterized in that** the following steps are carried out:
1) coating of the inert nucleus by spraying with a single aqueous or hydroalcoholic suspension-solution, which includes the active ingredient, the alkaline reacting compound and the pharmaceutically acceptable excipient or excipients;
2) drying the active layer formed in step 1;
3) coating of the charged nuclei by spraying a solution which contains the enteric coating polymer and the pharmaceutically acceptable excipient, in order to form the gastro-resistant external coating layer;
being all the steps performed in a single fluidized bed coater.

2. Procedure as claimed in claim 1, **characterized in that** after the step of coating of the charged nuclei, an additional drying is carried out.

3. Procedure as claimed in claim 1, **characterized in that** the pharmaceutically acceptable excipients present in the aqueous or hidroalcoholic solution-suspension contain a binder selected from a group which includes saccharose, starch, methylcellulose, CMC, HPC, HPMC, polyvinyl pyrrolidone (PVP), dextrine or gum arabic, either alone or mixed, dissolved in water, ethanol or a mixture of both at 50% (v/v).

4. Procedure as claimed in claim 1, **characterized in that** the alkaline reaction compound is selected from a group which includes trisodium phosphate, disodium phosphate, magnesium oxide, magnesium hydroxide, magnesium carbonate, aluminium hydroxide, carbonate, phosphate or citrate of aluminium, calcium, sodium or potassium, and the mixed compounds of aluminium/magnesium Al₂O₃.6MgO.CO₂.12H₂O or MgO.Al₂O₃.2SiO₂.nH₂O and amino acids with alkaline reaction.

5. Procedure as claimed in claim 1, **characterized in that** the pharmaceutically acceptable excipients present in the aqueous or hydroalcoholic solution-suspension contain a surface-active agent selected from a group which includes sodium lauryl sulphate, polysorbate, poloxamer or other ionic and non-ionic surface-active agents.

6. Procedure as claimed in claim 1, **characterized in that** the pharmaceutically acceptable excipients present in the aqueous or hydroalcoholic solution-suspension contain a filling material selected from a group which includes lactose, starch, saccharose and microcrystalline cellulose.

7. Procedure as claimed in claim 1, **characterized in that** the pharmaceutically acceptable excipients present in the aqueous or hydroalcoholic solution-suspension contain a disintegrating-swelling excipient selected from a group which includes starch, CMCCa, sodium glycolate starch and L-HPC.

8. Procedure as claimed in claim 1, **characterized in that** the enteric coating polymer present in the external gastro-resistant coating is selected from a group which includes methyl cellulose, HEC, HBC, HPMC, ethyl cellulose, HMC, HPC, polyoxyethylene glycol, castor oil, cellulose phthalic acetate, phthalate of HPMC, succinate acetate of HMC, sodium carboxymethylamylopectin, chitosan, alginic acid, carrageenans, galactomannons, tragacanth, shellac, agar-agar, gum arabic, guar gum, xanthan gum, polyacrylic, acids, methacrylics and their salts, PVA, polyethylene and polyproprylene oxides and mixtures thereof.

9. Procedure as claimed in claim 1, **characterized in that** the excipients present in the external gastro-resistant coating contain a plasticizer selected from a group which includes TEC, PEG, cetyl and stearyl alcohol.

10. Procedure as claimed in claim 1, **characterized in that** the excipients present in the external gastro-resistant coating contain a surface-active agent selected from a group which includes sodium lauryl sulphate, polysorbate and poloxamer.

11. Procedure as claimed in claim 1, **characterized in that** the excipients present in the external gastro-resistant coating contain a pigment selected from a group which includes titanium dioxide and iron sesquioxide.

12. Procedure as claimed in claim 1, **characterized in that** the excipients present in the external gastro-resistant coating contain a lubricant selected from a group which includes talc, magnesium stearate and glyceryl monostearate.

13. Oral pharmaceutical preparation obtainable by using the procedure of claims 1 to 12.

14. Oral pharmaceutical preparation as claimed in claim 13 **characterized in that** the inert nucleus is a neutral spherical microgranule which includes in its composition two or more of the following substances: sorbitol, manitol, saccharose, starch, microcrystalline cellulose, lactose, glucose, trehalose, maltitol or fructose.

15. Oral pharmaceutical preparation as claimed in claims 13 or 14 **characterized in that** the inert nucleus has an initial size between 200 and 1800 micrometres, preferably between 600-900 micrometres.

## Patentansprüche

1. Verfahren zur Herstellung eines oralen Arzneimittels, bestehend aus
a) einem inerten Kern;
b) einer löslichen aktiven Schicht oder einer Schicht, die sich schnell in Wasser zersetzt, hergestellt aus einer einzelnen wässrigen oder hydroalkoholischen Lösungs-Suspension, die folgendes beinhaltet:
- einen aktiven Bestandteil mit Aktivität gegen Geschwüre der allgemeinen Formel I 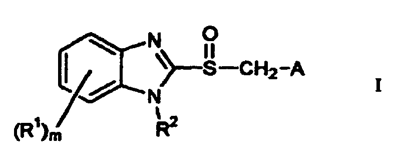
in welcher:
A sein kann: 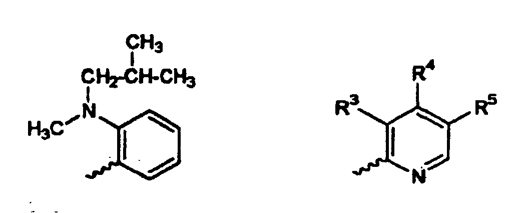
worin:
R³ und R⁵ gleich oder unterschiedlich sind und Wasserstoff, Alkyl, Alkoxy, oder Alkoxyalkoxy sein können;
R⁴ ist Wasserstoff, Alkyl, Alkoxy, welches wahlweise auch fluoriniert sein kann, Alkoxyalkoxy, oder Alkoxycycloalkyl;
R¹ ist Wasserstoff, Alkyl, Halogen, Cyano, Carboxy, Carboalkoxy, Carboalkoxyalkyl, Carbamoyl, Carbamoylalkyl, Hydroxy, Alkoxy, Hydroxyalkyl, Trifluormethyl, Acyl, Carbamoyloxy, Nitro, Acyloxy, Aryl, Aryloxy, Alkylthio oder Alkylsulfinyl;
R² ist Wasserstoff, Alkyl, Acyl, Carboalkoxy, Carbamoyl, Alkylcarbamoyl, Dialkylcarbamoyl, Alkylcarbonylmethyl, Alkoxycarbonylmethyl oder Alkylsulfonyl; und
m ist eine ganze Zahl von 0 bis 4;
oder der Formel II oder III, 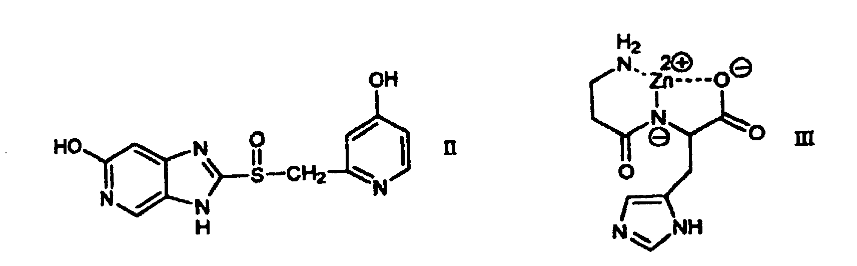
- eine alkalisch reagierende Verbindung und
- mindestens einen pharmazeutisch annehmbaren Arzneimittelträger der Gruppe, die enthält: ein Bindemittel, eine oberflächenaktive Substanz, ein Füllmaterial und ein unter Auflösung anschwellender Hilfsstoff;
und
c) einer magenresistenten äußeren Beschichtung, hergestellt aus einer Lösung, die folgendes beinhaltet:
- ein magensaftresistentes Beschichtungspolymer; und
- mindestens einen Hilfsstoff aus der Gruppe, die enthält: einen Weichmacher, eine oberflächenaktive Substanz, ein Pigment und ein Gleitmittel,
wobei das Verfahren **dadurch gekennzeichnet ist, dass** die folgenden Schritte ausgeführt werden:
1) Beschichten des inerten Kerns durch Besprühen mit einer einzelnen wässrigen oder hydroalkoholischen Lösungs-Suspension, die den aktiven Bestandteil, die alkalisch reagierende Verbindung und den pharmazeutisch annehmbaren Hilfsstoff oder Hilfsstoffe beinhaltet;
2) Trocknen der im ersten Schritt gebildeten aktiven Schicht;
3) Beschichten der beladenen Kerne durch Besprühen mit einer Lösung, die das magensaftresistente Beschichtungspolymer und den pharmazeutisch annehmbaren Hilfsstoff enthält, um die magenresistente äußere Beschichtungsschicht herzustellen;
wobei alle Schritte in einer einzigen Fließbettbeschichtungsmaschine durchgeführt werden.

2. Verfahren nach Anspruch 1, **dadurch gekennzeichnet, dass** nach der Beschichtung der beladenen Kerne ein zusätzlicher Trocknungsschritt durchgeführt wird.

3. Verfahren nach Anspruch 1, **dadurch gekennzeichnet, dass** die pharmazeutisch annehmbaren Hilfsstoffe, die in der wässrigen oder hydroalkoholischen Lösungs-Suspension vorliegen, ein Bindemittel enthalten, das aus einer Gruppe, die Saccharose, Stärke, Methylzellulose, CMC, HPC, HPMC, Polyvinylpyrrolidon (PVP), Dextrin oder Gummi Arabicum, entweder einzeln oder als Gemisch, aufgelöst in Wasser, Ethanol oder in einer 50%-igen (v/v) Mischung daraus, enthält, ausgewählt wird.

4. Verfahren nach Anspruch 1, **dadurch gekennzeichnet, dass** die alkalisch reagierende Verbindung aus einer Gruppe, die Trinatriumphosphat, Dinatriumphosphat, Magnesiumoxid, Magnesiumhydroxid, Magnesiumcarbonat, Aluminiumhydroxid, Carbonat, Phosphat oder Citrat von Aluminium, Kalzium, Natrium oder Kalium, und die gemischten Verbindungen von Aluminium/Magnesium Al₂O₃.6MgO.CO₂.12H₂O oder MgO.Al₂O₃.2SiO₂.nH₂O und Aminosäuren mit alkalischer Reaktion enthält, ausgewählt wird.

5. Verfahren nach Anspruch 1, **dadurch gekennzeichnet, dass** die pharmazeutisch annehmbaren Hilfsstoffe, die in der wässrigen oder hydroalkoholischen Lösungs-Suspension vorliegen, ein oberflächenaktives Mittel aus einer Gruppe, die Natriumlaurylsulfat, Polysorbat, Poloxamer oder andere ionische oder nichtionische oberflächenaktive Substanzen enthält, beinhalten.

6. Verfahren nach Anspruch 1, **dadurch gekennzeichnet, dass** die pharmazeutisch annehmbaren Hilfsstoffe, die in der wässrigen oder hydroalkoholischen Lösungs-Suspension vorliegen, ein Füllmaterial aus einer Gruppe, die Laktose, Stärke, Saccharose und mikrokristalline Zellulose enthält, beinhalten.

7. Verfahren nach Anspruch 1, **dadurch gekennzeichnet, dass** die pharmazeutisch annehmbaren Hilfsstoffe, die in der wässrigen oder hydroalkoholischen Lösungs-Suspension vorliegen, einen unter Zersetzung anschwellenden Hilfsstoff aus einer Gruppe, die Stärke, CMCCa, Natriumglykolatstärke und L-HPC enthält, beinhalten.

8. Verfahren nach Anspruch 1, **dadurch gekennzeichnet, dass** das magensaftresistente Beschichtungspolymer, das in der äußeren magenresistenten Beschichtung vorliegt, aus einer Gruppe ausgewählt wird, die folgende Stoffe beinhaltet: Methylzellulose, HEC, HBC, HPMC, Ethylzellulose, HMC, HPC, Polyoxyethylenglykol, Rizinusöl, Zellulosephthalsäureacetat, HPMC-Phthalat, HMC-Succinat-Acetat, Natriumcarboxymethylamylopectin, Chitosan, Alginsäure, Carrageene, Galactomannone, Tragant, Schellack, Agar-Agar, Gummi Arabicum, Guar Gum, Xanthanlösung, Polyacrylsäuren, Methacrylsäuren und ihre Salze, PVA, Polyethylen- und Polypropylenoxide und deren Mischungen.

9. Verfahren nach Anspruch 1, **dadurch gekennzeichnet, dass** die Hilfsstoffe, die in der äußeren magenresistenten Beschichtung vorliegen, einen Weichmacher enthalten, der aus einer Gruppe ausgewählt wird, die folgende Stoffe enthält: TEC, PEG, Cetyl- und Stearylalkohol.

10. Verfahren nach Anspruch 1, **dadurch gekennzeichnet, dass** die Hilfsstoffe, die in der äußeren magenresistenten Beschichtung vorliegen, eine oberflächenaktive Substanz enthalten, die aus einer Gruppe ausgewählt wird, die folgende Stoffe enthält: Natriumlaurylsulfat, Polysorbat und Poloxamer.

11. Verfahren nach Anspruch 1, **dadurch gekennzeichnet, dass** die Hilfsstoffe, die in der äußeren magenresistenten Beschichtung vorliegen, ein Pigment enthalten, das aus einer Gruppe ausgewählt wird, die Titandioxid und Eisensesquioxid enthält.

12. Verfahren nach Anspruch 1, **dadurch gekennzeichnet, dass** die Hilfsstoffe, die in der äußeren magenresistenten Beschichtung vorliegen, einen Gleitstoff enthalten, der aus einer Gruppe ausgewählt wird, die Talk, Magesiumstearat und Glycerylmonostearat einschliesst.

13. Orales Arzneimittel, das durch die Anwendung des Verfahrens der Ansprüche 1 bis 12 erhalten werden kann.

14. Orales Arzneimittel nach Anspruch 13, **dadurch gekennzeichnet, dass** der inerte Kern ein neutrales rundes Mikrokörnchen ist, das in seiner Zusammensetzung zwei oder mehr der folgenden Substanzen enthält: Sorbitol, Manitol, Saccharose, Stärke, mikrokristalline Zellulose, Laktose, Glukose, Trehalose, Maltit oder Fruktose.

15. Orales Arzneimittel nach den Ansprüchen 13 oder 14, **dadurch gekennzeichnet, dass** der inerte Kern einen Anfangsdurchmesser zwischen 200 und 1800 µm, bevorzugt zwischen 600 und 900 µm hat.

## Revendications

1. Procédé de fabrication d'une préparation pharmaceutique orale comprenant:
a) un noyau inerte ;
b) une couche active soluble ou une couche qui se désintègre rapidement dans de l'eau, préparée à partir d'une suspension-solution aqueuse ou hydroalcoolique unique qui comprend:
- un ingrédient actif ayant une activité anti-ulcéreuse de formule générale I : 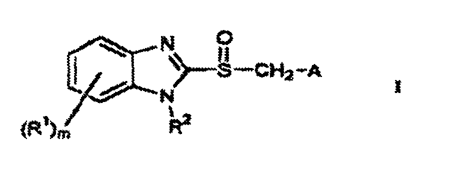
dans laquelle
A peut être 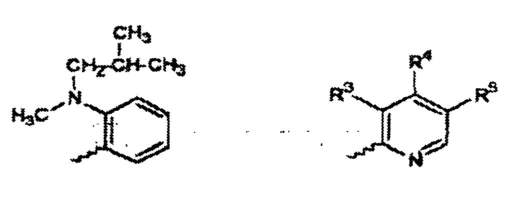
où :
R³ et R⁵ sont identiques ou différents, et peuvent être un atome d'hydrogène, un groupe alkyle, un groupe alcoxy ou un groupe alcoxyalcoxy ;
R⁴ est un atome d'hydrogène, un groupe alkyle, un groupe alcoxy qui peut être éventuellement fluoré, un groupe alcoxyalcoxy ou un groupe alcoxycycloalkyle ;
R¹ est un atome d'hydrogène, un groupe alkyle, un atome d'halogène, un groupe cyano, un groupe carboxy, un groupe carboalcoxy, un groupe carboalcoxyalkyle, un groupe carbamoyle, un groupe carbamoylalkyle, un groupe hydroxy, un groupe alcoxy, un groupe hydroxyalkyle, un groupe trifluorométhyle, un groupe acyle, un groupe carbamoyloxy, un groupe nitro, un groupe acyloxy, un groupe aryle, un groupe aryloxy, un groupe alkylthio ou un groupe alkylsulfinyle ;
R² est un atome d'hydrogène, un groupe alkyle, un groupe acyle, un groupe carboalcoxy, un groupe carbamoyle, un groupe alkylcarbamoyle, un groupe dialkylcarbamoyle, on groupe alkylcarbonylméthyle, un groupe alcoxycarbonylméthyle ou un groupe alkylsulfonyle ; et
m est un nombre entier de 0 à 4 ;
ou de formule II ou III : 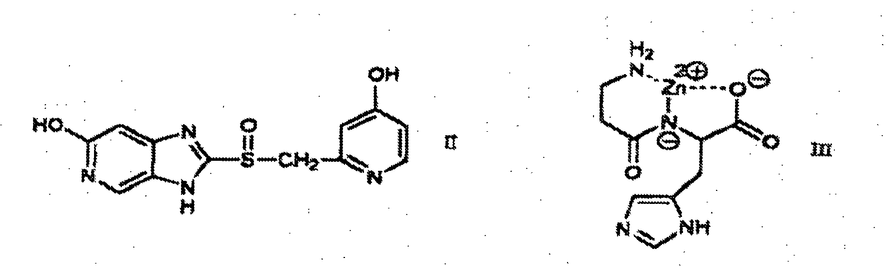
- un composé réactionnel alcalin
et
- au moins un excipient pharmaceutiquement acceptable choisi dans le groupe qui comprend un liant, un tensioactif, un matériau de remplissage et un excipient de désintégration-gonflement ; et
c) un revêtement externe gastro-résistant préparé à partir d'une solution qui comprend :
- un polymère de revêtement entérique ; et
- au moins un excipient choisi dans le groupe qui comprend un plastifiant, un tensioactif, un pigment et un lubrifiant,
le procédé étant **caractérisé en ce que** les étapes suivantes consistent à :
1) enduire le noyau inerte par pulvérisation avec une seule solution-suspension aqueuse ou hydroalcoolique qui comprend l'ingrédient actif, le composé réactionnel alcalin et le ou les excipients pharmaceutiquement acceptables ;
2) sécher la couche active formée dans l'étape 1 ;
3) enduire les noyaux chargés par pulvérisation d'une solution qui comprend le polymère de revêtement entérique et l'excipient pharmaceutiquement acceptable, afin de former la couche de revêtement externe gastro-résistante ;
toutes les étapes étant réalisées dans un seul appareil à enduire en lit fluidisé.

2. Procédé selon la revendication 1, **caractérisé en ce qu'**après l'étape de revêtement des noyaux chargés, un séchage supplémentaire est réalisé.

3. Procédé selon la revendication 1, **caractérisé en ce que** les excipients pharmaceutiquement acceptables présents dans la solution-suspension aqueuse ou hydroalcoolique comprennent un liant choisi dans le groupe comprenant le saccharose, l'amidon, la méthylcellulose, la CMC, l'HPC, l'HPMC, la polyvinyipyrrolidone (PVP), la dextrine ou la gomme arabique, seul ou mélangé, dissous dans de l'eau, de l'éthanol ou un mélange des deux à 50 % (volume/volume).

4. Procédé selon la revendication 1, **caractérisé en ce que** le composé réactionnel alcalin choisi dans le groupe comprenant le phosphate de trisodium, le phosphate de disodium, l'oxyde de magnésium, l'hydroxyde de magnésium, le carbonate de magnésium, l'hydroxyde d'aluminium, le carbonate, le phosphate ou le citrate d'aluminium, de calcium, de sodium ou de potassium, et des composés d'aluminium/magnésium mélangés Al₂O₃.6 MgO,CO₂,12 H₂O ou MgO.Al₂O₃.2 SiO₂.n H₂O et d'acides aminés avec une réaction alcaline.

5. Procédé selon la revendication 1, **caractérisé en ce que** les excipients pharmaceutiquement acceptables présents dans la solution-suspension aqueuse ou hydroalcoolique comprennent un tensioactif choisi dans le groupe comprenant le laurylsulfate de sodium, le polysorbate, le poloxamère ou d'autres tensioactifs ioniques et non ioniques.

6. Procédé selon la revendication 1, **caractérisé en ce que** les excipients pharmaceutiquement acceptables présents dans la solution-suspension aqueuse ou hydroalcoolique comprennent un matériau de remplissage choisi dans le groupe comprenant le lactose, l'amidon, le saccharose et la cellulose microcristalline.

7. Procédé selon la revendication 1, **caractérisé en ce que** les excipients pharmaceutiquement acceptables présents dans la solution-suspension aqueuse ou hydroalcoolique comprennent un excipient de désintégration-gonflement choisi dans le groupe comprenant l'amidon, le CMCCa, l'amidon de glycolate de sodium et la L-HPC.

8. Procédé selon la revendication 1, **caractérisé en ce que** le polymère de revêtement entérique présent dans le revêtement externe gastro-résistant est choisi dans le groupe comprenant la méthylcellulose, l'HEC, l'HBC, l'HPMC, l'éthylcellulose, l'HMC, l'HPC, le polyoxyéthylène glycol, l'huile de ricin, l'acétate phtalique de cellulose, le phtatate d'HPMC, le succinate-acétate d'HMC, la carboxyméthylamylopectine sodique, la chitosane, l'acide alginique, les carragénines, les galactomannanes, la gomme adragante, la gomme laque, l'agar-agar, la gomme arabique, la gomme de guar, la gomme xanthique, les acides polyacryliques, les acides méthacryliques et leurs sels, le PVA, les oxydes de polyéthylène et de polypropylène et des mélanges de ceux-ci.

9. Procédé selon la revendication 1, **caractérisé en ce que** les excipients présents dans le revêtement externe gastro-résistant comprennent un plastifiant choisi dans le groupe comprenant le TEC, le PEG, l'alcool cétylique et l'alcool stéarylique.

10. Procédé selon la revendication 1, **caractérisé en ce que** les excipients présents dans le revêtement externe gastro-résistant comprennent un tensioactif choisi dans le groupe comprenant le laurylsulfate de sodium, le polysorbate et le poloxamère.

11. Procédé selon la revendication 1, **caractérisé en ce que** les excipients présents dans le revêtement externe gastro-résistant comprennent un pigment choisi dans le groupe comprenant le dioxyde de titane et le sesquioxyde de fer.

12. Procédé selon la revendication 1, **caractérisé en ce que** les excipients présents dans le revêtement externe gastro-résistant comprennent un lubrifiant choisi dans le groupe comprenant le talc, le stéarate de magnésium et le monostéarate de glycéryle.

13. Préparation pharmaceutique orale pouvant être obtenue en utilisant le procédé des revendications 1 à 2.

14. Préparation pharmaceutique orale selon la revendication 13, **caractérisée en ce que** le noyau inerte est un microgranule sphérique neutre qui comprend dans sa composition deux des substances suivantes ou plus : le sorbitol, le manitol, le saccharose, l'amidon, la cellulose microcristalline, le lactose, le glucose, le tréhalose, le maltitol ou le fructose.

15. Préparation pharmaceutique orale selon la revendication 13 ou 14, **caractérisée en ce que** le noyau inerte a une taille initiale comprise dans la plage allant de 200 à 1800 micromètres, de préférence allant de 600 à 900 micromètres.
